(19) Europäisches Patentamt
European Patent Office
Office européen des brevets

(11) Veröffentlichungsnummer: **0 051 196**
**B1**

# (12) EUROPÄISCHE PATENTSCHRIFT

(45) Veröffentlichungstag der Patentschrift: **09.11.83**

(21) Anmeldenummer: **81108487.0**

(22) Anmeldetag: **19.10.81**

(51) Int. Cl.³: **C 07 C 93/14**, C 07 D 307/52, C 07 D 333/20, A 01 N 33/10, A 01 N 43/06

**(54) 4-Benzyloxy-alpha-trichlormethyl-benzylamine, ein Verfahren zu ihrer Herstellung, sie enthaltende Schädlingsbekämpfungsmittel, ihre Herstellung und Verwendung.**

(30) Priorität: **30.10.80 DE 3040912**

(43) Veröffentlichungstag der Anmeldung: **12.05.82 Patentblatt 82/19**

(45) Bekanntmachung des Hinweises auf die Patenterteilung: **09.11.83 Patentblatt 83/45**

(84) Benannte Vertragsstaaten: **AT BE CH DE FR GB IT LI NL**

(56) Entgegenhaltungen:
**DE - A - 2 301 397**
**FR - A - 2 358 104**

(73) Patentinhaber: **BAYER AG, Zentralbereich Patente, Marken und Lizenzen, D-5090 Leverkusen 1, Bayerwerk (DE)**

(72) Erfinder: **Schulze, Andreas, Dr., Volswinkler Strasse 35, D-5060 Bergisch Gladbach 2 (DE)**
Erfinder: **Sasse, Klaus, Dr., Pützweg 13, D-5060 Bergisch Gladbach (DE)**
Erfinder: **Naumann, Klaus, Dr., Richard-Wagner-Strasse 9, D-5090 Leverkusen (DE)**
Erfinder: **Roessler, Peter, Dr., Elsterstrasse 15, D-5060 Bergisch-Gladbach (DE)**
Erfinder: **Zoebelein, Gerhard, Dr., Domblick 46, D-5090 Leverkusen 31 (DE)**

**EP 0 051 196 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 4-Benzyloxy-α-trichlormethyl-benzylamine, ein Verfahren zu ihrer Herstellung, sie enthaltende Schädlingsbekämpfungsmittel, ihre Herstellung und Verwendung

Die vorliegende Erfindung betrifft neue 4-Benzyloxy-α-trichlormethyl-benzylamine, ein Verfahren zu ihrer Herstellung, ihre Verwendung als Schädlingsbekämpfungsmittel, vorzugsweise gegen Arthropoden, sie enthaltende Schädlingsbekämpfungsmittel und ihre Verwendung.

Bestimmte 4-Formyl- bzw. 4-Dialkylamino-α-trichlormethylbenzylamine sind als Zwischenprodukte und als Stoffe mit nicht näher erläuterter Wirkung gegen Viren, Tumoren, Pilze und Insekten bekannt geworden (Tetrahedron 1967, 23, Seiten 1713 – 1721; Britische Patentschrift 1 173 677). Ihre Wirkung ist jedoch in der Praxis – vor allem bei niederen Aufwandmengen – nicht ausreichend.

Ferner ist bekannt geworden, bestimmte 4,4'-disubstituierte α-Trichlormethylbenzyl-aniline als Insektizide zu verwenden (Deutsche Offenlegungsschrift 2 301 397). Auch die insektizide Wirkung dieser Stoffe ist jedoch vor allem bei niedrigen Aufwandmengen nicht befriedigend.

Weiterhin ist es bekannt geworden, bestimmte Diphenyletherderivate zur Bekämpfung schädlicher Insekten zu verwenden. Doch ist die Wirkung dieser Verbindungen bei einigen Anwendungsarten vor allem bei niedrigen Aufwandkonzentrationen nicht voll befriedigend (Deutsche Offenlegungsschrift 2 418 343).

Darüber hinaus werden in der FR-A-2 358 104 bestimmte Benzyloxy-benzylamine als Insektizide beschrieben, deren Benzylgruppe in α-Stellung des Benzylaminoteils einen Methylrest enthalten kann. Die insektizide Wirksamkeit dieser vorbekannten Verbindungen ist jedoch insbesondere bei niedrigen Aufwandmengen nicht voll befriedigend.

Gegenstand der Erfindung sind die neuen 4-Benzyloxy-α-trichlormethyl-benzylamine der allgemeinen Formel I

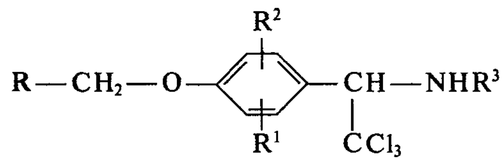

(I)

in welcher

R für einen gegebenenfalls substituierten Phenylrest steht,

$R^1$ und $R^2$ gleich oder verschieden sind und für Wasserstoff, Halogen oder für gegebenenfalls substituiertes Alkyl oder Alkoxy stehen und

$R^3$ für Alkyl, Cycloalkyl, Alkenyl oder Alkinyl steht, wobei diese Reste substituiert sein können,

und deren Salze.

Gegenstand der Erfindung ist weiterhin ein Verfahren zur Herstellung der neuen 4-Benzyloxy-α-trichlormethylbenzylamine der allgemeinen Formel I, und ihrer Salze, welches dadurch gekennzeichnet ist, daß man Verbindungen der allgemeinen Formel II

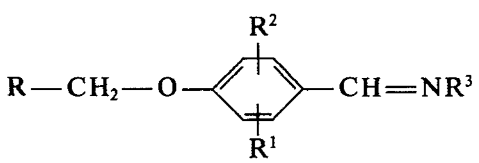

(II)

in welcher

R, $R^1$, $R^2$ und $R^3$ die oben angegebene Bedeutung haben,

mit Trichloressigsäure, gegebenenfalls in Gegenwart eines Lösungsmittels, umsetzt und gegebenenfalls durch weitere Umsetzung mit einer Säure die Salze herstellt.

Der gegebenenfalls substituierte Phenylrest R kann einen oder mehrere, gleiche oder verschiedene Substituenten enthalten.

Bevorzugt steht der gegebenenfalls substituierte Phenylrest R für einen Rest der Formel Ia

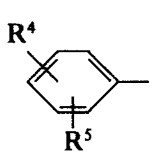

(Ia)

in welcher

$R^4$ und $R^5$ gleich oder verschieden sind und für Wasserstoff, Halogen, Nitro und Cyano oder für gegebenenfalls substituiertes Alkyl, Alkoxy oder Alkylthio stehen.

Vorzugsweise steht einer der Reste $R^4$ und $R^5$ in der 3- oder 4-Stellung, insbesondere in der 4-Stellung. Insbesondere steht $R^4$ für Wasserstoff. Besonders bevorzugt stehen $R^4$ für Wasserstoff und $R^5$ für einen Substituenten in der 4-Stellung. Alkyl, Alkoxy und Alkylthio $R^4$ und $R^5$ sind geradkettig oder verzweigt und enthalten vorzugsweise 1 bis 8, insbesondere 1 bis 4 und ganz besonders bevorzugt 1 oder 2 Kohlenstoffatome. Diese Reste Alkyl, Alkoxy und Alkylthio können einen oder mehrere, vorzugsweise 1 bis 5, insbesondere 1 bis 3 gleiche oder verschiedene Substituenten enthalten, wobei bevorzugt als Substituenten Halogen, wie Fluor, Chlor, Brom und Jod, vorzugsweise Fluor und Chlor stehen. Als Alkyl, Alkoxy und Alkylthio sowie Halogenalkyl, Halogenalkoxy und Halogenalkylthio seien beispielhaft genannt: Methyl, Ethyl, n- und i-Propyl und n-, i-, s- und t-Butyl, Chlormethyl, Chlordifluormethyl, Trifluormethyl, 1,1-Difluor-2-dichlorethyl, Trifluormethoxy, 1,1-Difluor-2,2-dichlorethoxy, Chlormethylthio, Trifluormethylthio und 1,1-Difluor-2,2-dichlormethylthio.

Halogen $R^4$ und $R^5$ bedeutet Fluor, Chlor, Brom und Jod, vorzugsweise Fluor, Chlor und Brom, insbesondere Fluor und Chlor.

Als besonders bevorzugte substituierte Phenylreste R seien genannt: 4-Methylphenyl, 4-Trifluormethylphenyl, 4-Methoxyphenyl, 4-Trifluormethoxyphenyl, 4-Trifluormethylthiophenyl und 4-Chlor- und 4-Fluorphenyl ($R^4$ steht für Wasserstoff und $R^5$ steht für 4-Methyl, 4-Trifluormethyl, 4-Methoxy, 4-Trifluormethoxy, 4-Trifluormethylthio, 4-Chlor und 4-Fluor).

Die Substituierten $R^1$ und $R^2$ können die verschiedenen freien Positionen im Phenylring des Benzylamin-Teils besetzen. Vorzugsweise befindet sich einer der Reste $R^1$ und $R^2$ in 3-Stellung. Bevorzugt steht einer der Reste $R^1$ und $R^2$ für Wasserstoff und besonders bevorzugt sind beide Reste $R^1$ und $R^2$ Wasserstoff. Gegebenenfalls substituiertes Alkyl und Alkoxy $R^1$ und $R^2$ sind geradkettig oder verzweigt und enthalten vorzugsweise 1 bis 5, insbesondere 1 bis 3 und ganz besonders bevorzugt 1 oder 2 Kohlenstoffatome. Als Substituent dieser Reste kommt vor allem Halogen, wie Fluor, Chlor, Brom und Jod, insbesondere Fluor und Chlor in Frage. Beispielhaft seien genannt: Methyl, Ethyl, n- und i-Propyl, Methoxy, Ethoxy und Trifluormethoxy. Halogen $R^1$ und $R^2$ bedeutet Fluor, Chlor, Brom und Jod, vorzugsweise Fluor, Chlor und Brom.

Besonders bevorzugt sind Verbindungen der Formel I, in welcher einer der Reste $R^1$ und $R^2$ für Halogen oder Alkoxy steht oder beide Reste $R^1$ und $R^2$ für Halogen stehen. Ganz besonders bevorzugt sind Verbindungen der Formel I in welcher $R^1$ und $R^2$ für Wasserstoff stehen.

Der gegebenenfalls substituierte Alkylrest $R^3$ ist geradkettig oder verzweigt und enthält vorzugsweise 1 bis 18, insbesondere 1 bis 6 und ganz besonders bevorzugt 1 bis 4 Kohlenstoffatome. Beispielhaft seien aufgeführt Methyl, Ethyl, n- und i-Propyl, n-, i-, s- und t-Butyl.

Der gegebenenfalls substituierte Cycloalkylrest $R^3$ enthält vorzugsweise 3 bis 7, insbesondere 3, 5 oder 6 Ringkohlenstoffatome. Es kann außer durch die unten genannten Substituenten auch durch Alkyl mit vorzugsweise 1 bis 4 Kohlenstoffatomen, vorzugsweise durch Methyl substituiert sein. Beispielhaft seien Cyclopropyl, Cyclopentyl, Cyclohexyl und Methylcyclohexyl genannt.

Gegebenenfalls substituiertes Alkenyl und Alkinyl $R^3$ ist geradkettig oder verzweigt und enthält vorzugsweise 3 bis 8, insbesondere 3 bis 6 Kohlenstoffatome, wobei diese Reste vorzugsweise 1 Doppel- bzw. Dreifachbindung enthalten. Beispielhaft seien Allyl, Propargyl und 1,1-Diethyl-propargyl genannt.

Die gegebenenfalls substituierten Alkyl-, Alkenyl- und Alkinylreste $R^3$ können einen oder mehrere, vorzugsweise 1 bis 3, insbesondere 1 Substituenten tragen. Hierbei seien als bevorzugte Substituenten, Halogen, wie Fluor, Chlor, Brom und Jod, Hydroxy, Cyano, Alkoxy, Alkylthio oder ein heterocyclischer Ring genannt. Alkoxy und Alkylthio enthalten hierbei vorzugsweise 1 bis 5 insbesondere 1 oder 2 Kohlenstoffatome. Beispielhaft seien Methoxy, Ethoxy, Methylthio und Ethylthio aufgeführt.

Der heterocyclische Ring als Substituent der obigen Reste $R^3$, vorzugsweise von $R^3$ als Alkyl, insbesondere Methyl, enthält vorzugsweise 5 bis 7, insbesondere 5 oder 6 Ringglieder und 1 bis 3, vorzugsweise 1 oder 2 gleiche oder verschiedene Heteroatome, wie Stickstoff, Schwefel und Sauerstoff. Er kann gesättigt sein oder bis 3 Doppelbindungen enthalten und aromatischen Charakter aufweisen. Der heterocyclische Ring kann auch beispielsweise durch Alkyl, wie Methyl oder Halogen, wie Chlor substituiert sein. Beispielhaft seien Pyridyl, Imidazolyl, Thiazolyl und vorzugsweise Thienyl und Furyl genannt.

Besonders bevorzugt steht $R^3$ in Formel I für gegebenenfalls durch Alkoxy mit 1 oder 2 Kohlenstoffatomen substituiertes Alkyl mit 1 bis 18 Kohlenstoffatomen, für Alkyl mit 1 bis 4 Kohlenstoffatomen (vorzugsweise Methyl) welches durch Furyl (2) oder Thienyl (2) substituiert ist, für Cyclohexyl oder Methylcyclohexyl oder für Alkenyl oder Alkinyl mit 3 bis 6 Kohlenstoffatomen.

Als erfindungsgemäße Verbindungen werden solche der Formel I bevorzugt, in welcher R für den Rest der Formel Ia

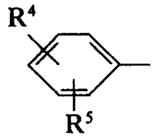

(I a)

steht, in welcher

$R^4$ und $R^5$ gleich oder verschieden sind und für Wasserstoff, Halogen, Nitro und Cyano oder für gegebenenfalls substituiertes Alkyl, Alkoxy oder Alkylthio stehen und
$R^1$ für Wasserstoff oder Halogen steht und
$R^2$ für Wasserstoff, Halogen oder Alkoxy steht und
$R^3$ für Alkyl, welches durch Alkoxy, Furyl oder Thienyl substituiert sein kann oder für Cycloalkyl, Alkylcycloalkyl oder für Alkenyl oder Alkinyl steht

und deren Salze.

Als erfindungsgemäße Verbindungen werden solche der Formel I besonders bevorzugt, in welchen R für den Rest der Formel Ia

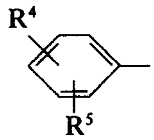

(I a)

steht, in welcher

$R^4$ und $R^5$ gleich oder verschieden sind und für Wasserstoff, Halogen, Nitro und Cyano oder für gegebenenfalls substituiertes Alkyl, Alkoxy oder Alkylthio mit jeweils 1–4 Kohlenstoffatomen im Alkylteil stehen und
$R^1$ für Wasserstoff oder Halogen steht und
$R^2$ für Wasserstoff, Halogen oder Alkoxy mit 1–5 Kohlenstoffatomen steht und
$R^3$ für Alkyl mit 1–18 Kohlenstoffatomen oder für Alkyl mit 1–4 Kohlenstoffatomen, welches durch Alkoxy mit 1–5 Kohlenstoffatomen, Furyl oder Thienyl substituiert sein kann oder für Cycloalkyl, Methylcycloalkyl oder für Alkenyl oder Alkinyl mit 3–8 Kohlenstoffatomen steht

und deren Salze.

Unter Salzen der Verbindungen der Formel I sind die üblichen, pflanzenverträglichen Salze mit anorganischen Säuren (z. B. Halogenwasserstoffsäuren, wie HCl, HBr, HJ, Salpetersäure), z. B. die Chloride, Bromide, Jodide oder Nitrate und mit organischen Säuren (z. B. Oxalsäure, $CX_3COOH$ mit X = F, Cl, Br), z. B. die Oxalate oder Trihalogenacetate (Halogen = F, Cl, Br) zu verstehen.

Unter den Verbindungen der Formel I und ihrer Salze werden sowohl die Racemate als auch die optisch aktiven Stereoisomere, welche nach den allgemein üblichen Trennmethoden erhältlich sind, verstanden.

Im einzelnen seien folgende erfindungsgemäße Verbindungen genannt:

(4-Benzyloxy-α-trichlormethyl-benzyl)-methylamin
(4-Benzyloxy-α-trichlormethyl-benzyl)-ethylamin
(4-Benzyloxy-α-trichlormethyl-benzyl)-n-propylamin
(4-Benzyloxy-α-trichlormethyl-benzyl)-i-propylamin
(4-Benzyloxy-α-trichlormethyl-benzyl)-n-butylamin
(4-Benzyloxy-α-trichlormethyl-benzyl)-i-butylamin
(4-Benzyloxy-α-trichlormethyl-benzyl)-furfurylamin
(4-Benzyloxy-α-trichlormethyl-benzyl)-thienylmethylamin
1-(4-Benzyloxy-α-trichlormethyl-benzyl)-3-ethoxy-propylamin
[4-(4'-Chlor-benzyloxy)-α-trichlormethyl-benzyl]-ethylamin
[4-(4'-Chlor-benzyloxy)-α-trichlormethyl-benzyl]-n-propylamin
[4-(4'-Chlor-benzyloxy)-α-trichlormethyl-benzyl]-i-propylamin
[4-(4'-Chlor-benzyloxy)-α-trichlormethyl-benzyl]-n-butylamin
[4-(4'-Chlor-benzyloxy)-α-trichlormethyl-benzyl]-i-butylamin
[4-(4'-Chlor-benzyloxy)-α-trichlormethyl-benzyl]-n-hexylamin
[4-(4'-Chlor-benzyloxy)-α-trichlormethyl-benzyl]-furfurylamin
[4-(4'-Chlor-benzyloxy)-α-trichlormethyl-benzyl]-thienylmethylamin
[4-(4'-Methyl-benzyloxy)-α-trichlormethyl-benzyl]-methylamin
[4-(4'-Methyl-benzyloxy)-α-trichlormethyl-benzyl]-ethylamin

[4-(4'-Methyl-benzyloxy)-α-trichlormethyl-benzyl]-n-propylamin
[4-(4'-Methyl-benzyloxy)-α-trichlormethyl-benzyl]-i-propylamin
[4-(4'-Methyl-benzyloxy)-α-trichlormethyl-benzyl]-allylamin
[4-(4'-Methyl-benzyloxy)-α-trichlormethyl-benzyl]-propargylamin
[4-(4'-Methyl-benzyloxy)-α-trichlormethyl-benzyl]-n-butylamin
[4-(4'-Methyl-benzyloxy)-α-trichlormethyl-benzyl]-i-butylamin
[4-(4'-Methyl-benzyloxy)-α-trichlormethyl-benzyl]-sec.-butylamin
[4-(4'-Methyl-benzyloxy)-α-trichlormethyl-benzyl]-tert.-butylamin
[4-(4'-Methyl-benzyloxy)-α-trichlormethyl-benzyl]-n-hexylamin
[4-(4'-Methyl-benzyloxy)-α-trichlormethyl-benzyl]-2-ethylhexylamin
[4-(4'-Methyl-benzyloxy)-α-trichlormethyl-benzyl]-n-dodecylamin
[4-(4'-Methyl-benzyloxy)-α-trichlormethyl-benzyl]-cyclohexylamin
[4-(4'-Methyl-benzyloxy)-α-trichlormethyl-benzyl]-furfurylamin
[4-(4'-Methyl-benzyloxy)-α-trichlormethyl-benzyl]-thienylmethylamin
1-[4-(4'-Methyl-benzyloxy)-α-trichlormethyl-benzyl]-3-ethoxy-propylamin
[4-(4'-Methoxy-benzyloxy)-α-trichlormethyl-benzyl]-methylamin
[4-(4'-Methoxy-benzyloxy)-α-trichlormethyl-benzyl]-ethylamin
[4-(4'-Methoxy-benzyloxy)-α-trichlormethyl-benzyl]-i-propylamin
[4-(4'-Methoxy-benzyloxy)-α-trichlormethyl-benzyl]-n-butylamin
[4-(4'-Methoxy-benzyloxy)-α-trichlormethyl-benzyl]-i-butylamin
[4-(4'-Methoxy-benzyloxy)-α-trichlormethyl-benzyl]-sec.-butylamin
[4-(4'-Methoxy-benzyloxy)-α-trichlormethyl-benzyl]-n-hexylamin
[4-(4'-Methoxy-benzyloxy)-α-trichlormethyl-benzyl]-furfurylamin
[4-(4'-Methoxy-benzyloxy)-α-trichlormethyl-benzyl]-thienylmethylamin
1-[4-(4'-Methoxy-benzyloxy)-α-trichlormethyl-benzyl]-3-ethoxy-propylamin
[4-(4'-Methyl-benzyloxy)-3-methoxy-α-trichlormethyl-benzyl]-n-butylamin
[4-(4'-Methyl-benzyloxy)-3-methoxy-α-trichlormethylbenzyl]-i-butylamin
[4-(4'-Methyl-benzyloxy)-3-methoxy-α-trichlormethylbenzyl]-furfurylamin
[4-(4'-Methyl-benzyloxy)-3-methoxy-α-trichlormethylbenzyl]-thienylmethylamin
[4-(4'-Methoxy-benzyloxy)-3-methoxy-α-trichlormethyl-benzyl]-n-butylamin
[4-(4'-Methoxy-benzyloxy)-3-methoxy-α-trichlormethyl-benzyl]-furfurylamin
[4-(4'-Methoxy-benzyloxy)-3-methoxy-α-trichlormethyl-benzyl]-thienylmethylamin
[4-(4'-Fluor-benzyloxy)-α-trichlormethyl-benzyl]-n-butylamin
[4-(4'-Fluor-benzyloxy)-α-trichlormethyl-benzyl]-furfurylamin
[4-(4'-Trifluormethyl-benzyloxy)-α-trichlormethyl-benzyl]-i-butylamin
[4-(4'-Trifluormethyl-benzyloxy)-α-trichlormethyl-benzyl]-n-hexylamin
[4-(4'-Trifluormethyl-benzyloxy)-α-trichlormethylbenzyl]-furfurylamin
[4-(3'-Trifluormethyl-4'-chlor-benzyloxy)-α-trichlormethyl-benzyl]-i-butylamin
[4-(4'-Trifluormethoxy-benzyloxy)-α-trichlormethyl-benzyl]-i-propylamin
[4-(4'-Trifluormethoxy-benzyloxy)-α-trichlormethylbenzyl]-n-butylamin
[4-(4'-Trifluormethoxy-benzyloxy)-α-trichlormethylbenzyl]-i-butylamin
[4-(4'-Trifluormethoxy-benzyloxy)-α-trichlormethyl-benzyl]-n-hexylamin
[4-(4'-Trifluormethoxy-benzyloxy)-α-trichlormethyl-benzyl]-furfurylamin
[4-(4'-Trifluormethoxy-benzyloxy)-α-trichlormethylbenzyl]-thienylmethylamin
[4-(4'-Trifluormercapto-benzyloxy)-α-trifluormethyl-benzyl]-n-butylamin
[4-(4'-Trifluormercapto-benzyloxy)-α-trifluormethyl-benzyl]-furfurylamin
sowie deren Chloride und Trichloracetate.

Der Reaktionsablauf zur Herstellung der neuen erfindungsgemäßen Wirkstoffe kann durch folgendes Formelschema erläutert werden, wenn 1-[4-(4'-Methylbenzyloxy)-benzyliden]-3-ethoxy-propylamin und Trichloressigsäure als Ausgangsstoffe dienen

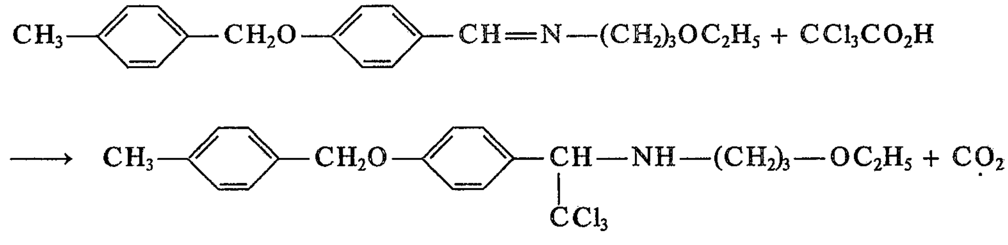

Die für die Herstellung der α-Trichlormethylamine der allgemeinen Formel I benötigten Schiff'schen Basen der allgemeinen Formel II sind zum größten Teil bekannt (Deutsche Offenlegungsschrift 2 631 948) oder können nach bekannten Verfahren dargestellt werden (vgl. Houben–Weyl, Methoden der organischen Chemie, Band VII/1 Jahrgang 1954, Seiten 454 bis 458 und Band XI/2 Jahrgang 1958,

Seiten 74 bis 85, Thieme Verlag, Stuttgart).

Als Lösungsmittel für das erfindungsgemäße Verfahren kommen alle inerten organischen Lösungsmittel, z. B. gegebenenfalls halogenierte aliphatische und aromatische Kohlenwasserstoffe wie Cyclohexan, Benzol, Toluol, Xylol, Chlorbenzol, Tetrachlorkohlenstoff usw. in Frage. Das erfindungsgemäße Verfahren kann auch ohne Lösungsmittel durchgeführt werden, jedoch sind die Ausbeuten in diesem Fall meist schlechter. Vorzugsweise werden Toluol oder Xylol als Lösungsmittel verwendet.

Zweckmäßigerweise werden die Ausgangsstoffe bei Temperaturen von 80 bis 130° C, vorzugsweise bei 90 bis 110° C zusammengegeben und die Reaktion auch bei diesen Temperaturen durchgeführt.

Die Umsetzung erfolgt im allgemeinen bei Normaldruck.

Bei der Durchführung des erfindungsgemäßen Verfahrens setzt man auf 1 Mol Schiff'sche Base vorzugsweise 1 bis 3 Mol, insbesondere 1 bis 2 Mol Trichloressigsäure ein.

Der Verlauf des erfindungsgemäßen Verfahrens kann anhand der $CO_2$-Entwicklung auf einfache Weise verfolgt werden.

Die Aufarbeitung des Reaktionsgemisches erfolgt nach allgemein bekannten Methoden. Zweckmäßigerweise wird das Lösungsmittel abdestilliert und gewünschtenfalls das erhaltene Produkt z. B. aus Petrolether umkristallisiert.

Die Salze können nach den allgemein üblichen Salzbildungsmethoden aus den Verbindungen der Formel I und entsprechenden anorganischen oder organischen Säuren, wie Halogenwasserstoffsäuren (HCl, HBr, HJ), Salpetersäure oder organischen Säuren, wie Oxalsäure und Trihalogenessigsäuren ($CX_3COOH$; $X = F$, Cl, Br) erhalten werden.

Die neuen Verbindungen der Formel I und ihre Salze sind bei geringer Warmblütertoxizität in überraschender Weise sehr gut wirksam gegen Arthropoden, insbesondere Insekten, wobei sie auch eine gute Wirkung als Eisterilantien aufweisen. Im folgenden sollen unter erfindungsgemäßen »Wirkstoffen« jeweils die Verbindungen der Formel I und ihre Salze verstanden werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dystercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa

decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp. Aus der Ordnung der Diptera z. B. Aedes spp., Anopheles spp., Culex spp., Drosophila melanogaster, Musca spp., Fannia spp., Caliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u. a.

Die erfindungsgemäßen Wirkstoffe können ferner in ihren handelsüblichen Formulierungen sowie in den aus diesen Formulierungen bereiteten Anwendungsformen in Mischung mit Synergisten

vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Wirksamkeit der erfindungsgemäßen Wirkstoffe, soll anhand der folgenden Beispiele erläutert werden:

## Beispiel A

### Aedes-Test

| | |
|---|---|
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Man füllt die wäßrigen Wirkstoffzubereitungen in Gläser und setzt anschließend etwa 20 Mückenlarven (Aedes aegypti) in jedes Glas ein.

Nach der gewünschten Zeit wird die Abtötung in % bestimmt. Dabei bedeutet 100%, daß alle Tiere abgetötet wurden; 0% bedeutet, daß keine Tiere abgetötet wurden.

Bei einem beispielhaft mit einer Wirkstoffkonzentration von 0,0001% durchgeführten Versuch zeigten bei diesem Test, z. B. die folgenden erfindungsgemäßen Verbindungen nach 14 Tagen einen Abtötungsgrad von 100%: Verbindungen der Herstellungsbeispiele 18, 28, 30, 34, 35, 36, 40, 42, 57, 58 und 72.

## Beispiel B

### Plutella-Test

| | |
|---|---|
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea) werden durch Sprühen mit der Wirkstoffzubereitung der gewünschten Konzentration behandelt. Nach Antrocknen der Spritzbrühe wird von der behandelten Pflanze ein Blatt entnommen, in eine Plastikdose gelegt und mit Raupen der Kohlschabe (Plutella maculipennis) besetzt. Nach 2 und 4 Tagen wird jeweils ein weiteres Blatt von derselben Pflanze für die Nachfütterung verwendet.

Nach der gewünschten Zeit wird die Abtötung in % bestimmt. Dabei bedeutet 100%, daß alle Tiere abgetötet wurden; 0% bedeutet, daß keine Tiere abgetötet wurden.

Bei einem beispielhaft mit einer Wirkstoffkonzentration von 0,01% durchgeführten Versuch zeigten bei diesem Test, z. B. die folgenden erfindungsgemäßen Verbindungen nach 14 Tagen einen Abtötungsgrad von 100%: Verbindungen der Herstellungsbeispiele 1, 2, 3, 4, 17, 18, 27, 28, 30, 33, 34, 35, 36, 39, 40, 42, 51, 56, 57, 58, 70 und 72.

Die Herstellung der erfindungsgemäßen Verbindungen soll anhand der folgenden Herstellungsbeispiele erläutert werden (alle %Angaben beziehen sich auf Gewichtsprozente und alle Temperaturangaben sind °C).

Beispiel 1

N-[4-(4′-Methyl-benzyloxy)-α-trichlormethyl-benzyl]-furfurylamin

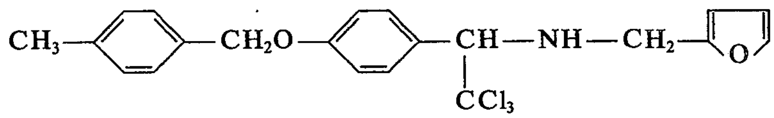

a) Trichloressigsäure wird an der Ölpumpen unter Feuchtigkeitsausschluß in einen gewogenen Kolben destilliert und anschließend mit soviel trockenem Toluol versetzt, daß 1 g Lösung 0,6 g Trichloressigsäure enthält.

b) Zu 91,5 g (0,3 Mol) [4-(4′-Methyl-benzyloxy)-benzyliden]-furfurylamin in 300 ml trockenem Toluol werden bei 100° C 114 g (0,42 Mol) der unter a) hergestellten Lösung von Trichloressigsäure so getropft, daß die $CO_2$-Entwicklung nicht zu heftig wird. Anschließend wird noch 2 Stunden bei 100° C gerührt, abgekühlt, mit Wasser gewaschen, getrocknet, eingedampft und das Rohprodukt aus Petrolether umkristallisiert.
Ausbeute 86 g = 68% der Theorie
Schmelzpunkt 80° C

Beispiel 2

[4-(4′-Methyl-benzyloxy)-α-trichlormethyl-benzyl]-n-butylamin

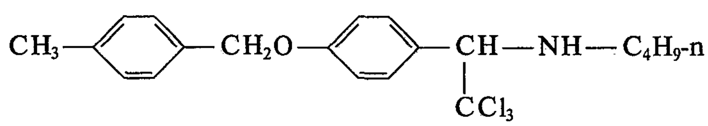

a) [4-(4′-Methyl-benzyloxy)-benzyliden]-n-butylamin

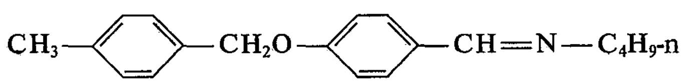

2 Mol 4-Methyl-benzyloxy-benzaldehyd und 2,6 Mol n-Butylamin werden in 1 Liter Toluol mit 0,5 g p-Toluolsulfonsäure am Wasserabscheider so lange unter Rückfluß erhitzt, bis die Wasserabscheidung beendet ist. Anschließend wird das Toluol im Vakuum eingedampft und das Rohprodukt im Vakuum destilliert.
$Kp_{0,1}$ 215 – 220° C Schmelzpunkt 43° C
Ausbeute 505 g = 90% der Theorie

b) 1 Mol der unter a) erhaltenen Schiff'schen Base wird mit 2 Mol Trichloressigsäure in trockenem Toluol analog Beispiel 1b) umgesetzt. Man erhält 366 g [4-(4′-Methyl-benzyloxy)-α-trichlormethyl-benzyl]-n-butylamin = 91% der Theorie, als gelbes Öl $n_D^{20}$ 1,5619

Beispiel 3

[4-(4′-methyl-benzyloxy)-α-trichlormethyl-benzyl]-ethylamin

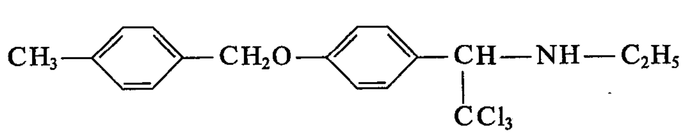

a) [4-(4′-Methyl-benzyloxy)-benzyliden]-ethylamin

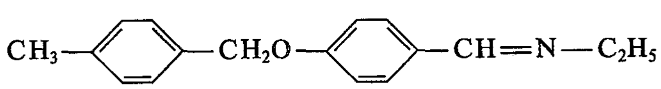

Zu 120 ml 50%iger Ethylaminlösung in Wasser und 600 ml Toluol werden 70 g $Na_2SO_4$ wasserfrei gegeben und 30 Minuten gerührt, abgesaugt, die Lösung zu einer Mischung aus 113 g (0,5 Mol) 4-Methyl-benzyloxy-benzaldehyd und 60 g $Na_2SO_4$ wasserfrei gegeben, 10 Stunden bei Raumtemperatur gerührt, abgesaugt, eingedampft und im Vakuum destilliert.
$Kp_{0,15}$ 195° C Schmelzpunkt 67° C

Ausbeute 105 g = 90% der Theorie

b) Zu 93,6 g (0,4 Mol) der Schiff'schen Base aus 3a) in 400 ml trockenem Toluol werden 145 g Trichloressigsäure in 250 ml trockenem Toluol bei 100° C schnell zugetropft, 4 Stunden bei 100° C gerührt, abgekühlt, mit Wasser gewaschen, getrocknet, eingedampft, und aus Petrolether umkristallisiert,
Schmelzpunkt 103 – 104° C
Ausbeute 128 g = 86% der Theorie an [4-(4'-Methylbenzyloxy)-α-trichlormethyl-benzyl]-ethylamin

## Beispiel 4

### Salz der Trichloressigsäure mit [4-(4'-Methyl-benzyloxy) – α-trichlormethyl-benzyl]-ethylamin

Man gibt zu einer Lösung von 1 Mol [4-(4'-Methyl-benzyloxy)-α-trichlormethyl-benzyl]-ethylamin in Toluol eine Lösung von 1 Mol Trichloressigsäure in Toluol und dampft das Toluol im Vakuum ein.
Ausbeute 525 g = 98% der Theorie
Schmelzpunkt 126° C

Analog können die folgenden Verbindungen der Tabelle 1 dargestellt werden:

Tabelle 1

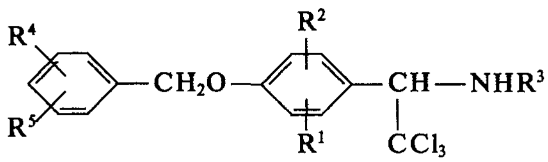

| Bsp. Nr. | $R^4$ | $R^5$ | $R^1$ | $R^2$ | $R^3$ | Schmelzpunkt (°C) oder Brechungs-index ($n_D^{20}$) |
|---|---|---|---|---|---|---|
| 5 | H | H | H | H | $-CH_3$ | |
| 6 | H | H | H | H | $-C_4H_9n$ | |
| 7 | H | H | H | H | $-C_4H_9t$ | 150 |
| 8 | H | H | H | H | $-C_6H_{13}n$ | 81 |
| 9 | H | H | H | H | 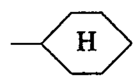 | 117 |
| 10 | H | H | H | H | $-(CH_2)_3-OC_2H_5$ | 85 |
| 11 | H | H | H | H | $-CH_2-$ 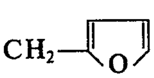 | |
| 12 | H | H | H | H | $-CH_2-$ 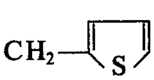 | |
| 13 | H | 4-Cl | H | H | $-C_3H_7i$ | |
| 14 | H | 4-Cl | H | H | $-C_4H_9n$ | |
| 15 | H | 4-Cl | H | H | $-C_4H_9i$ | |
| 16 | H | 4-Cl | H | H | $-C_6H_{13}n$ | 1,5641 |

Fortsetzung

| Bsp. Nr. | $R^4$ | $R^5$ | $R^1$ | $R^2$ | $R^3$ | Schmelzpunkt (°C) oder Brechungs-index ($n_D^{20}$) |
|---|---|---|---|---|---|---|
| 17 | H | 4-Cl | H | H | $-CH_2-CH-C_2H_5$<br>\|<br>$C_4H_9n$ | 1,5543 |
| 18 | H | 4-Cl | H | H | $-CH_2-$ 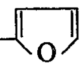 | 1,5958 |
| 19 | H | 4-Cl | H | H | $-CH_2-$ 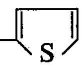 | |
| 20 | 3-Cl | 4-Cl | H | H | $-C_4H_9n$ | |
| 21 | 3-Cl | 4-Cl | H | H | $-C_{12}H_{25}n$ | 1,5480 |
| 22 | 3-Cl | 4-Cl | H | H | $-C_{14}H_{29}n$ | 1,5408 |
| 23 | 3-Cl | 4-Cl | H | H | 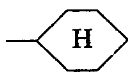 | 1,5717 |
| 24 | 3-Cl | 4-Cl | 3-Br | 5-Br | $C_4H_9s$ | 1,587 |
| 25 | H | $4-CH_3$ | H | H | $CH_3$ | |
| 26 | H | $4-CH_3$ | H | H | $-C_3H_7n$ | |
| 27 | H | $4-CH_3$ | H | H | $-C_3H_7i$ | 106 |
| 28 | H | $4-CH_3$ | H | H | $-CH_2-CH=CH_2$ | 51 |
| 29 | H | $4-CH_3$ | H | H | $-CH_2-C\equiv CH$ | |
| 30 | H | $4-CH_3$ | H | H | $-C_4H_9i$ | 65—66 |
| 31 | H | $4-CH_3$ | H | H | $-C_4H_9t$ | |
| 32 | H | $4-CH_3$ | H | H | $CH_3$<br>\|<br>$-C-C_2H_5$<br>\|<br>$CH_3$ | 102 |
| 33 | H | $4-CH_3$ | H | H | $-C_6H_{13}n$ | 1,5541 |
| 34 | H | $4-CH_3$ | H | H | $C_2H_5$<br>\|<br>$-C-C\equiv CH$<br>\|<br>$C_2H_5$ | 91 |
| 35 | H | $4-CH_3$ | H | H | $-CH_2-CH-C_2H_5$<br>\|<br>$C_4H_9n$ | 1,5528 |
| 36 | H | $4-CH_3$ | H | H | $-C_{12}H_{25}n$ | 38 |

Fortsetzung

| Bsp. Nr. | $R^4$ | $R^5$ | $R^1$ | $R^2$ | $R^3$ | Schmelzpunkt (°C) oder Brechungs-index ($n_D^{20}$) |
|---|---|---|---|---|---|---|
| 37 | H | 4-$CH_3$ | H | H | $-C_{14}H_{29}n$ | 52 |
| 38 | H | 4-$CH_3$ | H | H | $-C_{16}H_{33}n$ | 56 |
| 39 | H | 4-$CH_3$ | H | H | $-C_{18}H_{37}n$ | 58 |
| 40 | H | 4-$CH_3$ | H | H | 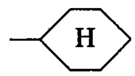 | 68—70 |
| 41 | H | 4-$CH_3$ | H | H | 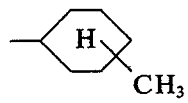 | 41—44 |
| 42 | H | 4-$CH_3$ | H | H | $-(CH_2)_3OC_2H_5$ | 1,5561 |
| 43 | H | 4-$CH_3$ | H | H | $-CH_2-$ 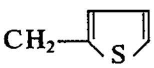 | |
| 44 | H | 3-$CH_3$ | H | H | $-C_4H_9s$ | 1,5583 |
| 45 | H | 3-$CH_3$ | H | H | $-C_4H_9i$ | |
| 46 | H | 3-$CH_3$ | H | H | $-C_6H_{13}n$ | 1,5516 |
| 47 | H | 3-$CH_3$ | H | H | $-(CH_2)_3OC_2H_5$ | 1,5461 |
| 48 | H | 4-$CH_3$ | H | 3-$OCH_3$ | $CH_3$ | |
| 49 | H | 4-$CH_3$ | H | 3-$OCH_3$ | $C_2H_5$ | |
| 50 | H | 4-$CH_3$ | H | 3-$OCH_3$ | $-C_4H_9s$ | 54—57 |
| 51 | H | 4-$CH_3$ | H | 3-$OCH_3$ | $-CH_2-$ 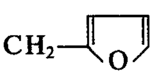 | 1,5842 |
| 52 | H | 4-$OCH_3$ | H | H | $-CH_3$ | |
| 53 | H | 4-$OCH_3$ | H | H | $-C_2H_5$ | |
| 54 | H | 4-$OCH_3$ | H | H | $-C_3H_7n$ | |
| 55 | H | 4-$OCH_3$ | H | H | $-C_4H_9i$ | |
| 56 | H | 4-$OCH_3$ | H | H | $-C_6H_{13}n$ | 1,5559 |
| 57 | H | 4-$OCH_3$ | H | H | $-(CH_2)_3OC_2H_5$ | 1,5582 |
| 58 | H | 4-$OCH_3$ | H | H | $-CH_2-$ 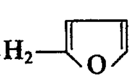 | 78—84 |
| 59 | H | 4-$OCH_3$ | H | H | $-CH_2-$ 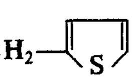 | |

Fortsetzung

| Bsp. Nr. | $R^4$ | $R^5$ | $R^1$ | $R^2$ | $R^3$ | Schmelzpunkt (°C) oder Brechungs-index ($n_D^{20}$) |
|---|---|---|---|---|---|---|
| 60 | H | 4-$OCH_3$ | H | 3-$OCH_3$ | $-C_4H_9i$ | |
| 61 | H | 4-$OCH_3$ | H | 3-$OCH_3$ | $-CH_2-$ 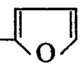 | 1,58 |
| 62 | H | 4-$OC_4H_9n$ | H | H | $-C_4H_9i$ | 1,5505 |
| 63 | H | 4-$CF_3$ | H | H | $-C_4H_9n$ | |
| 64 | 3-$CF_3$ | 4-Cl | H | H | $-CH_2-$ 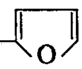 | |
| 65 | H | 4-$SCF_3$ | H | H | $-C_4H_9i$ | |
| 66 | H | 4-$SCF_3$ | H | H | $-CH_2-$ 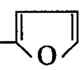 | |
| 67 | H | 4-F | H | H | $-C_4H_9n$ | |
| 68 | H | 4-$OCF_3$ | H | H | $-C_4H_9n$ | |
| 69 | H | 4-$OCF_3$ | H | H | $-C_4H_9i$ | |
| 70 | H | 4-$OCF_3$ | H | H | $-C_4H_9s$ | 1,5242 |
| 71 | H | 4-$OCF_3$ | H | H | $-C_6H_{13}n$ | 1,5203 |
| 72 | H | 4-$OCF_3$ | H | H | $-CH_2-$ 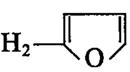 | 1,5432 |
| 73 | H | 4-$OCF_3$ | H | H | $-CH_2-$ 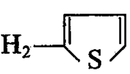 | |
| 74 | H | 4-$NO_2$ | H | H | $-C_4H_9n$ | |
| 75 | H | 4-$NO_2$ | H | H | $-C_6H_{13}n$ | 69 |
| 76 | H | 4-$NO_2$ | H | H | $-(CH_2)_3OC_2H_5$ | 62 |
| 77 | H | 4-$CH_3$ | H | H | $-CH_2CF_3$ | |
| 78 | H | 4-$CH_3$ | H | H | $-CH_2CH_2-CF_3$ | |

**Patentansprüche**

1. 4-Benzyloxy-α-trichlormethyl-benzylamine der allgemeinen Formel I

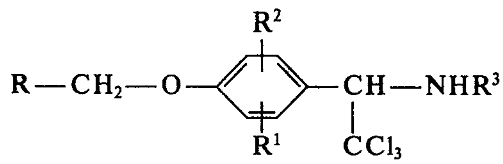

(I)

in welcher

R für einen gegebenenfalls substituierten Phenylrest steht,
$R^1$ und $R^2$ gleich oder verschieden sind und für Wasserstoff, Halogen oder für gegebenenfalls substituiertes Alkyl oder Alkoxy stehen und
$R^3$ für Alkyl, Cycloalkyl, Alkenyl oder Alkinyl steht, wobei diese Reste substituiert sein können,

und deren Salze.

2. Verfahren zur Herstellung von 4-Benzyloxy-α-trichlormethylbenzylaminen der allgemeinen Formel I und ihrer Salze

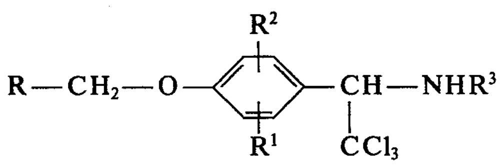

(I)

in welcher

R für einen gegebenenfalls substituierten Phenylrest steht,
$R^1$ und $R^2$ gleich oder verschieden sind und für Wasserstoff, Halogen oder für gegebenenfalls substituiertes Alkyl oder Alkoxy stehen und
$R^3$ für Alkyl, Cycloalkyl, Alkenyl oder Alkinyl steht, wobei diese Reste substituiert sein können,

dadurch gekennzeichnet, daß man Verbindungen der allgemeinen Formel II

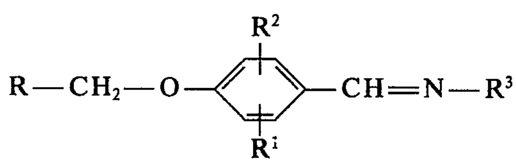

(II)

in welchen

R, $R^1$, $R^2$ und $R^3$ die oben angegebene Bedeutung haben,

mit Trichloressigsäure, gegebenenfalls in Gegenwart eines Lösungsmittels, umsetzt und gegebenenfalls durch weitere Umsetzung mit einer Säure die Salze herstellt.

3. 4-Benzyloxy-α-trichlormethyl-benzylamine gemäß Anspruch 1, in welchen

R für den Rest der Formel Ia

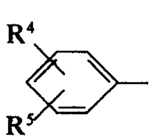

(Ia)

steht, in welcher

$R^4$ und $R^5$ gleich oder verschieden sind und für Wasserstoff, Halogen, Nitro und Cyano oder für gegebenenfalls substituiertes Alkyl, Alkoxy oder Alkylthio stehen und
$R^1$ für Wasserstoff oder Halogen steht und
$R^2$ für Wasserstoff, Halogen oder Alkoxy steht und
$R^3$ für Alkyl, welches durch Alkoxy, Furyl oder Thienyl substituiert sein kann oder für Cycloalkyl, Alkylcycloalkyl oder für Alkenyl oder Alkinyl steht.

4. 4-Benzyloxy-α-trichlormethyl-benzylamine gemäß Anspruch 1, in welchen

R für den Rest der Formel Ia

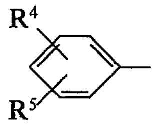

(Ia)

steht, in welcher

$R^4$ und $R^5$ gleich oder verschieden sind und für Wasserstoff, Halogen, Nitro und Cyano oder für gegebenenfalls substituiertes Alkyl, Alkoxy oder Alkylthio mit jeweils 1–4 Kohlenstoffatomen im Alkylteil stehen und
$R^1$ für Wasserstoff oder Halogen steht und
$R^2$ für Wasserstoff, Halogen oder Alkoxy mit 1–5 Kohlenstoffatomen steht und
$R^3$ für Alkyl mit 1–18 Kohlenstoffatomen oder für Alkyl mit 1–4 Kohlenstoffatomen, welches durch Alkoxy mit 1–5 Kohlenstoffatomen, Furyl oder Thienyl substituiert sein kann oder für Cycloalkyl, Methylcycloalkyl oder für Alkenyl oder Alkinyl mit 3–8 Kohlenstoffatomen steht.

5. 4-Benzyloxy-α-trichlormethyl-benzylamin der Formel

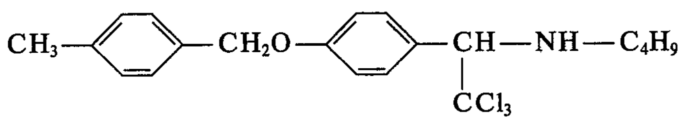

($C_4H_9$ = n $C_4H_9$ oder i $C_4H_9$)

und dessen Salze

6. 4-Benzyloxy-α-trichlormethyl-benzylamin der Formel

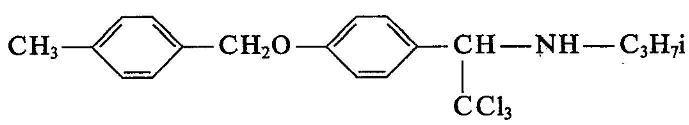

und dessen Salze.

7. 4-Benzyloxy-α-trichlormethyl-benzylamin der Formel

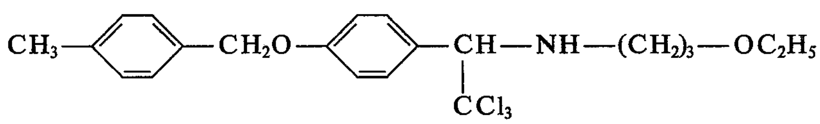

und dessen Salze.

8. Schädlingsbekämpfungsmittel, gekennzeichnet durch den Gehalt an mindestens einem 4-Benzyloxy-α-trichlormethyl-benzylamin der Formel I oder dessen Salz.

9. Verwendung von 4-Benzyloxy-α-trichlormethyl-benzylaminen der Formel I und deren Salzen zur Schädlingsbekämpfung.

10. Verfahren zur Herstellung von Schädlingsbekämpfungsmitteln, dadurch gekennzeichnet, daß man 4-Benzyloxy-α-trichlormethyl-benzylamine der Formel I und deren Salze mit Streckmitteln und/oder oberflächenaktiven Mitteln und gegebenenfalls sonstigen Formulierhilfsmitteln vermischt.

**Claims**

1. 4-Benzyloxy-α-trichloromethyl-benzylamines of the general formula I

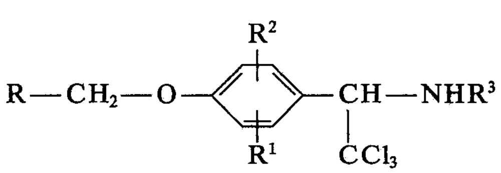

(I)

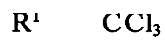

in which

R represents an optionally substituted phenyl radical,

$R^1$ and $R^2$ are identical or different and represent hydrogen, halogen or optionally substituted alkyl or alkoxy and

$R^3$ represents alkyl, cycloalkyl, alkenyl or alkinyl, it being possible for these radicals to be substituted,

and of their salts, characterised in that compounds of the general formula II

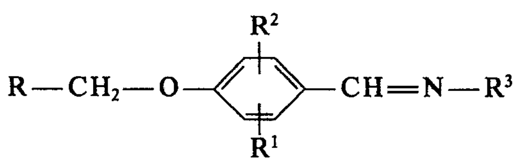

(II)

in which

R, $R^1$, $R^2$ and $R^3$ have the abovementioned meaning,

are reacted with trichloroacetic acid, if appropriate in the presence of a solvent, and, if desired, the salts are prepared by further reaction of the product with an acid.

3. 4-Benzyloxy-α-trichloromethyl-benzylamines according to Claim 1, in which

R represents the radical of the formula Ia

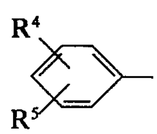

(Ia)

in which

$R^4$ and $R^5$ are identical or different and represent hydrogen, halogen, nitro, cyano or optionally substituted alkyl, alkoxy or alkylthio, and

$R^1$ represents hydrogen or halogen,

$R^2$ represents hydrogen, halogen or alkoxy and

$R^3$ represents alkyl, which can be substituted by alkoxy, furyl or thienyl, or cycloalkyl, alkylcycloalkyl, alkenyl or alkinyl.

4. 4-Benzyloxy-α-trichloromethyl-benzylamines according to Claim 1, in which

R represents the radical of the formula Ia

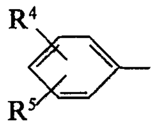

(Ia)

in which

$R^4$ and $R^5$ are identical or different and represent hydrogen, halogen, nitro, cyano or optionally substituted alkyl, alkoxy or alkylthio with in each case 1–4 carbon atoms in the alkyl part, and
$R^1$ represents hydrogen or halogen,
$R^2$ represents hydrogen, halogen or alkoxy with 1–5 carbon atoms and
$R^3$ represents alkyl with 1–18 carbon atoms, alkyl which has 1–4 carbon atoms and can be substituted by alkoxy with 1–5 carbon atoms, furyl or thienyl, or cycloalkyl, methylcycloalkyl or alkenyl or alkinyl with 3–8 carbons atoms.

5. The 4-benzyloxy-α-trichloromethyl-benzylamine of the formula

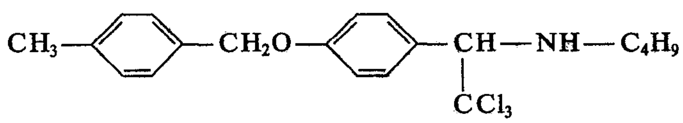

($C_4H_9$=n $C_4H_9$ or i $C_4H_9$

and its salts.

6. The 4-benzyloxy-α-trichloromethyl-benzylamine of the formula

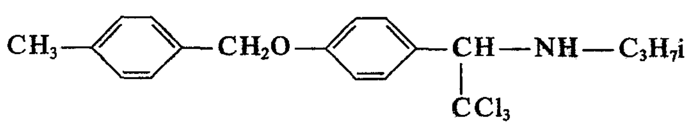

and its salts.

7. The 4-benzyloxy-α-trichloromethyl-benzylamine of the formula

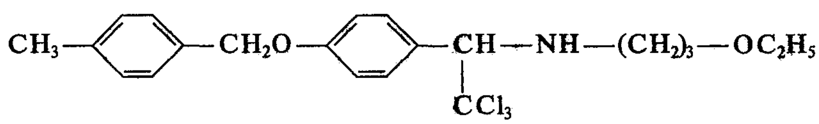

and its salts.

8. Pest-combating agents, characterised in that they contain at least one 4-benzyloxy-α-trichloromethyl-benzylamine of the formula I or a salt thereof.

9. Use of 4-benzyloxy-α-trichloromethyl-benzylamines of the formula I and its salts for combating pests.

10. Process for the preparation of pest-combating agents, characterised in that 4-benzyloxy-α-trichloromethyl-benzylamines of the formula I and salts thereof are mixed with extenders and/or surface-active agents and, if appropriate, other formulation auxiliaries.

**Revendications**

1. 4-Benzyloxy-α-trichlorométhyl-benzylamines de formule générale I

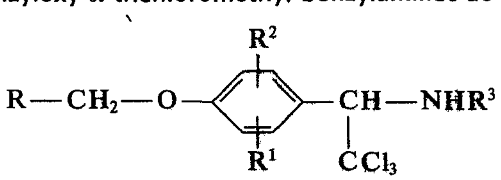

(I)

[dans laquelle

R représente un radical phényle éventuellement substitué,

$R^1$ et $R^2$ sont identiques ou différents et représentent un atome d'hydrogène ou d'halogène ou un groupe alkyle ou alcoxy éventuellement substitué, et

$R^3$ représente un groupe alkyle, cycloalkyle, alcényle ou alcynyle, ces radicaux pouvant être substitués],

et leurs sels.

2. Procédé de préparation de 4-benzyloxy-$\alpha$-trichlorométhyl-benzylamines de formule générale I

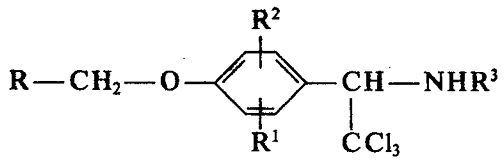

(I)

[dans laquelle

R représente un radical phényle éventuellement substitué,

$R^1$ et $R^2$ sont identiques ou différents et représentent un atome d'hydrogène ou d'halogène ou un groupe alkyle ou alcoxy éventuellement substitué; et

$R^3$ représente un groupe alkyle, cycloalkyle, alcényle ou alcynyle, ces radicaux puvant être substitués],

et de leurs sels, procédé procédé caractérisé en ce qu'on fait réagir des composés de formule générale II

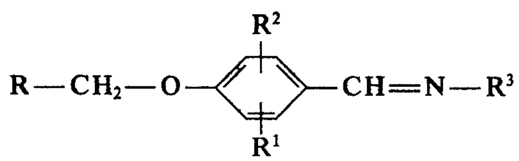

(II)

[dans laquelle

R, $R^1$, $R^2$ et $R^3$ ont le sens indiqué ci-dessus],

avec l'acide trichloracétique, éventuellement en présence d'un solvant, et l'on produit éventuellement les sels par une autre réaction avec un acide.

3. 4-Benzyloxy-$\alpha$-trichlorométhyl-benzylamines selon la revendication 1, dans lesquelles

R représente le radical de formule Ia

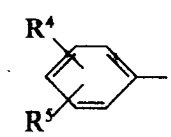

(I a)

dans laquelle

$R^4$ et $R^5$ sont identiques ou différents et représentent un atome d'hydrogène ou d'halogène ou un groupe nitro et cyano ou un groupe alkyle, alcoxy ou alkylthio éventuellement substitué, et

$R^1$ représente un atome d'hydrogène ou d'halogène, et

$R^2$ représente un atome d'hydrogène ou d'halogène ou un groupe alcoxy, et

$R^3$ représente un groupe alkyle (qui peut éventuellement être substitué par un groupe alcoxy, furyle ou thiényle) ou un radical cycloalkyle, alkylcycloalkyle ou alcényle ou alcynyle.

4. 4-Benzyloxy-$\alpha$-trichlorométhyl-benzylamines selon la revendication 1, dans lesquelles

R représente le radical de formule Ia

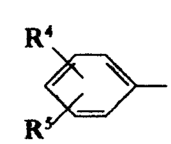

(I a)

dans laquelle

$R^4$ et $R^5$ sont identiques ou différents et représentent un atome d'hydrogène ou d'halogène, un groupe nitro et cyano ou un groupe alkyle, alcoxy ou alkylthio ayant chacun 1 à 4 atomes de carbone dans la partie alkyle éventuellement substituée, et

$R^1$ représente un atome d'hydrogène ou d'halogène, et

$R^2$ représente un atome d'hydrogène ou d'halogène ou un groupe alcoxy ayant 1 à 5 atomes de carbone, et

$R^3$ représente un groupe alkyle ayant 1 à 18 atomes de carbone ou un groupe ayant 1 à 4 atomes de carbone (qui peut être substitué par un groupe alcoxy ayant à 5 atomes de carbone, furyle ou thiényle) ou un groupe cycloalkyle, méthylcycloalkyle ou alcényle ou alcynyle ayant 3 à 8 atomes de carbone.

5. 4-Benzyloxy-α-trichlorométhyl-benzylamine de formule

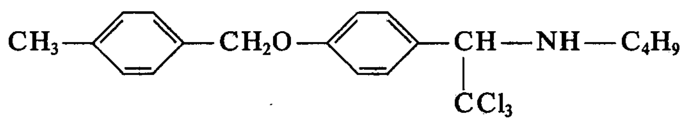

($C_4H_9$=n $C_4H_9$ ou i $C_4H_9$),

et ses sels.

6. 4-Benzyloxy-α-trichlorométhyl-benzylamine de formule

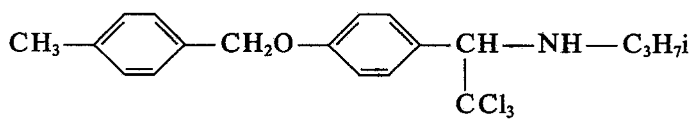

et ses sels.

7. 4-Benzyloxy-α-trichlorométhyl-benzylamine de formule

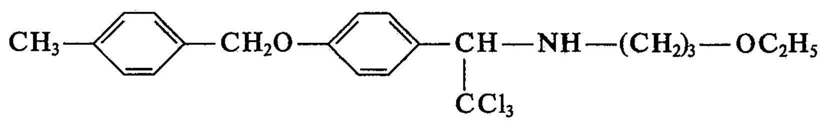

et ses sels.

8. Pesticide, caractérisé en ce qu'il contient au moins une 4-benzyloxy-α-trichlorométhyl-benzylamine de formule I ou son sel.

9. Application des 4-benzyloxy-α-trichlorométhylbenzylamines de formule I et de leurs sels à la lutte contre les parasites.

10. Procédé de préparation de pesticides, caractérisé en ce qu'on mélange des 4-benzyloxy-α-trichlorométhyl-benzylamines de formule I et leurs sels avec des agents d'allongement et/ou des agents tensio-actifs et éventuellement d'autres adjuvants de formulation.